(19) Europäisches Patentamt
European Patent Office
Office européen des brevets

(11) Publication number: 0 202 282 B1

# (12) EUROPEAN PATENT SPECIFICATION

(45) Date of publication of patent specification: **08.07.92**

(51) Int. Cl.⁵: **A61K 31/44**, A61K 37/02

(21) Application number: **85905809.1**

(22) Date of filing: **08.11.85**

(86) International application number:
**PCT/EP85/00597**

(87) International publication number:
**WO 86/02836 (22.05.86 86/11)**

The file contains technical information submitted after the application was filed and not included in this specification

## (54) NEW USE OF 1,4-DIHYDROPYRIDINE DERIVATIVES.

(30) Priority: **12.11.84 GB 8428552**

(43) Date of publication of application:
**26.11.86 Bulletin 86/48**

(45) Publication of the grant of the patent:
**08.07.92 Bulletin 92/28**

(84) Designated Contracting States:
**AT BE CH DE FR GB IT LI LU NL SE**

(56) References cited:
**EP-A- 150**
**EP-A- 125 803**
**DE-A- 2 210 667**
**DE-A- 3 015 219**
**FR-A- 2 528 431**
**EP-A- 117 888**
**BE-A- 886 259**
**DE-A- 2 335 466**
**FR-A- 2 528 425**
**US-A- 4 442 112**

**DRUGS OF THE FUTURE, vol. 8, no. 2, 1983; M.GRAU, pp. 136-137**

(73) Proprietor: **SANDOZ AG**
**Lichtstrasse 35**
**CH-4002 Basel(CH)**
(84) Designated Contracting States:
**BE CH FR GB IT LI LU NL SE**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

ACS SYMP. Ser., vol. 201, 1982; P.D.HENRY et al., pp. 175-184

CURR.CLIN.PRACT.SER., vol. 2, 1983, St.Louis, MO (US); P.D.HENRY, pp. 55-63

CURRENT THERAPEUTICS, vol. 24, no. 12, December 1983; M.J.RAND, pp. 13-23

CHEMICAL ABSTRACTS, vol. 100, no. 23, 04 June 1984, Columbus, OH (US); p. 63, no. 185788n

PERSPECT. CARDIOVASC. Res., vol. 9, chapter 20, 1984; P.D.HENRY, pp. 209-213

BIOCHEM. PHARMACOL., vol. 33, no. 14, 1984, Tokyo (JP); I.OHATA et al., pp. 2199-2205

LABORATORY INVESTIGATION, vol. 49, no. 2, 1983; R.GINSBURG et al., pp. 154-158

LIFE SCIENCES, vol. 32, 1983, Pergamon Press (US); F.V.DeFEUDIS, pp. 557-563

FED.PROC., vol. 44, no. 3, 1985; R.G.VAN VALEN et al., no. 1909

WO 84/02132

THE MERCK INDEX, 10th ed., 1983, Merck & Co., Rahway, NY (US); p. 226, no. 1611

INTERNISTISCHE WELT, vol. 2, no. 1, 1979; F.PAUL, pp. 1-8

CLIN.RES., vol. 27, no. 2, 1979; C.H.CHESNUT III et al., p. 229a

INTERNISTISCHE WELT, vol. 2, no. 7, 1979; E.KECK et al., pp. 253-261

SOUTHERN MEDICAL JOURNAL, vol. 77, no. 7, July 1984; A.W.DIDDLE et al., pp. 868-874

THE AMERICAN JOURNAL OF MEDICINE, vol. 84 (suppl. 3b), 25 March 1988; WEINSTEIN et al., pp. 102-108

BRITISH MEDICAL JOURNAL, vol. 2, no. 6186, 11 August 1979; pp. 364-365

CALCIF. TISSUE INT., vol. 27, Supp., no. 203, 1979; I.ZANZI et al., no. 203

(72) Inventor: **HOF, Robert, P.**
**Im Huebacher 12**
**CH-4460 Gelterkinden(CH)**
Inventor: **AZRIA, Moise**
**Bundesplatz 6**
**CH-4054 Basel(CH)**

## Description

This invention relates to the use of calcium antagonists. Such compounds are also called calcium entry blockers. Calcium antagonists represent a group of active substances which modulate entry of Ca (2+) ions through specific Ca (2+) channels in smooth muscles and are useful for the treatment of inter alia Angina pectoris, hypertension and for some products also migraine,

One group of calcium antagonists is characterised by a 1,4-dihydropyridine structure having an aryl or heterocylic group attached to the 4 position.

The invention relates to a new use of a compound of formula I

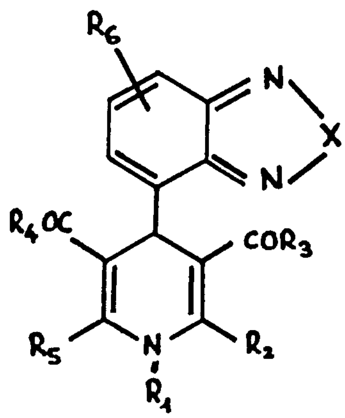

I

wherein

| | |
|---|---|
| $R_1$ | is hydrogen |
| $R_2$ and $R_5$ | independently are alkyl of 1 to 6 carbon atoms |
| $R_3$ and $R_4$ | independently are alkoxy of 1 to 6 carbon atoms |
| $R_6$ | is hydrogen |
| X | is oxygen |

in the preparation of a medicament suitable for use in the treatment of atherosclerosis.

Certain pharmacological activities of the compounds of formula I have been published in e.g. European Patent specification No. 150.

Particularly interesting compounds of this group of compounds include PY 108-068, i.e. darodipine, i.e. 4-(2,1,3-benzoxadiaxol-4-yl)-1,4-dihydro-2,6-dimethylpyridine-3,5-dicarboxylic acid diethyl ester, hereinafter referred to as PY and PN 200-110 i.e. 4-(2,1,3-benzoxadiazol-4-yl)-1,4-dihydro-2,6-dimethyl-3-methoxy carbonyl-pyridine-5-carboxylic acid isopropyl ester, hereinafter referred to as PN;

Calcium antagonists, e.g. a compound of formula I, modulate calcium ion transport systems at the cellular level and this has been extensively published in the literature. Movements at the cellular level are, however, very low and changes in the total levels of calcium ions in the blood would not be expected.

We have now found that ionized calcium levels in the blood are lowered to a significant extent by a compound of formula I over a long period of time. These compounds are therefore useful in the treatment of conditions related directly or indirectly to ionized calcium levels in the blood, particularly abnormal or elevated calcium levels in the blood, especially atherosclerosis

The compound of formula I preferably is PY but especially PN.

The effect of the compounds of formula I in lowering calcium levels is indicated in any standard animal test for hypercalcemic activity. In one test the compound is administered to rabbits (e.g. ca. 1 kg in weight) either as a suspension e.g. 0.1 to 5 mg (e.g. 0,1 mg) per ml of solution (i.e. blood plasma with ca. 1-5% alcohol) and 0.1-5 mg/kg animal body weight by intravenous infusion (0.5 ml per hour) or orally e.g. 1 to 5 mg/kg animal body weight as a suspension in distilled water. A significant lowering of calcium ions is observed using standard measuring techniques for calcium ions in the blood, e.g. using a calcium ion selective electrode, or by photometric methods.

One method for measuring calcium ion concentration is as follows:

The test involves continuous measurement of the ionized calcium concentration in the blood of young rabbits by means of a flow-through system containing $Ca^{2+}$-selective liquid membrane electrodes, based on the neutral carrier ETH 1001, inserted into an extracorporeal shunt established by means of indwelling catheters between the femoral artery and the femoral vein.

The blood is pumped from the artery to the vein, through the system containing the $Ca^{2+}$-selective electrodes, at a flow rate of 18 ml/h. A second peristaltic pump transports a reference electrolyte solution at a rate of 0.9 ml/h past a first reference electrode to a second electrode, known as the common electrode, where it meets and mixes with the circulating blood. The mixed fluids are then returned to the animal's circulatory system.

The concentration of ionized calcium in the blood is measured potentiometrically as follows: The difference in potential between the reference electrode and the $Ca^{2+}$-selective electrode is measured differentially relative to the common electrode. The difference is amplified and the analogue signal produced by the amplifier is processed by a digital voltmeter and continuously recorded as a function of time, the difference in voltage being proportional to the $Ca^{2+}$ concentration.

The ionized calcium concentration in blood (1.1-1.2 mmol/litre) is about $10^5$ higher than the detection limit of the electrodes in aqueous solutions of calcium ($10^{-5}$-$10^{-6}$ mmol/litre). The method is capable of detecting a change of 0.003 mmol/litre or 0.3% in the $Ca^{++}$ concentration.

The anti-atherogenic activity has been confirmed also by measurement of the inhibition of neointimal lesion development in the rat carotid artery (balloon catheterization test, essentially as published in A.W. Clowes et al., Lab. Invest. 49 [1983] 208-215).

In a first experiment a group of 12 rats was given PN 250 µg/kg/day in 10% ethanol-water s.c. A control group of 9 animals was non-injected and a further control group of 7 animals was vehicle-injected. Animals were randomized into 3 groups for catheterization and were weighed every other day throughout the study. Rats were anesthetized with ether inhalation.

The thoracic region was shaved and the external branch of the left common carotid artery was isolated by an incision from the lower mandible to the clavicle, followed by retracting the submaxillary gland (lateral) and several muscle bundles including the sternohyoideus (medial), sternomastoideus (lateral) and digastricus posterior belly (lateral). The external carotid was carefully separated for surrounding tissue and guy sutures were placed distal to the superior thyroid artery (cephalad end ligated) and proximal (end open) to the heart for retraction. Lidocaine was applied to vasodilate during catheter introduction. Following an incision with iris scissors, a Fogarty arterial embolectomy catheter (No. 2F) was inserted into the external branch of the carotid, passed into the aortic arch and slightly withdrawn to assure inflation of the catheter within the common carotid artery. The catheter was inflated using approximately 900 mmHg air pressure, drawn distally and deflated. This was repeated three times to assure complete de-endothelialization of the common carotid. The catheter was removed, the artery ligated and the incision closed. Under these conditions the carotid is completely de-endothelialized.

Animals were sacrificed 14 days after ballooning in the same sequence as the catheterization. Thirty minutes before fixation the animals were injected with a 2.25% Evan's Blue dye solution (1.5 mg/kg iv) to differentiate between the neointimal and re-endothelialied areas. Areas of the carotid that have endothelial regrowth exclude the dye and appear white. The remaining areas that lack endothelium are stained blue. Whole body (beating heart) perfusion fixations (90-110 mmHg) were then performed on anesthetized (sodium pentobarbital, 50.0 mg/kg ip) animals using 200 ml of 1% glutaraldehyde in 0.15 M sodium cacodylate buffer (pH 7.4, 37° C, 410 mOsm) followed by 400 ml of 3% glutaraldehyde in 0.15 M sodium cacodylate. The entry site of the fixative was a puncture in the left ventricle, with efflux collected by vacuum suction from the right atrium. The thoracic aorta flow was restricted to optimize carotid perfusion. Successful fixation was judged by upper body rigidity and an absence of blood from the large ateries.

In a second experiment the procedure as described above was followed, with groups consisting of non-injected controls (n = 8), animals treated with PY (n = 5) or PN (n = 8). Animals received drug for 2 days before catheterization and to the conclusion of the study (14 days). PY and PN were given at 1.0 mg/kg in 10% EtOH-water s.c. All animals (controls and drug-treated) were randomized into 3 groups for catheterization.

After each experiment the fixed carotid was removed and cut into 3 segments (distal, central, proximal). Tissue processing of only the central segment included 18 hours in 3% buffered glutaraldehyde, buffer rinsing, dehydration in an ascending ethanol series and infiltration with Spurr's resin. The samples were embedded with cross-sectional orientation so that sectioning (0.5 µm) would include blue areas which have not re-endothelialized and show continued proliferative responses. A two-step polychromatic stain utilizing toluidine blue and 1.0% basic fuchsin rendered histological differentiation to nuclear, cytoplasmic and extracellular connective matrix components.

All light microscopic histology slides were randomized, encoded and evaluated utilizing a Zeiss standard microscope and the Videoplan computerized image analyzer. Vessel measurements included maximum lesion height. Image calibration and magnification checks were performed for every group of slides analyzed. Analysis of groups was done using parametric and non-parametric statistical methods.

PN and PY were tolerated at all doses used, with control and treated animals showing similar weight gains. Maximal lesion height (µm) are tabulated in Table 1. Non-injected and s.c. vehicle controls (Expt. 1) gave equivalent values and have been combined for statistical purposes.

**TABLE 1**

**MAXIMAL INTIMAL HEIGHT (µm) FROM CONTROLS AND TREATED ANIMALS**

| | Dose (mg/kg) | Intima | p-Value |
|---|---|---|---|
| **Expt. 1** | | | |
| Controls n=16 | - | 84.9±21.3 | - |
| PN n=11 | 0.25 | 63.2±19.9 | p=0.006 |
| **Expt. 2** | | | |
| Controls n=8 | - | 72.4±17.4 | - |
| PY n=5 | 1.0 | 58.6±18.1 | p=0.097 |
| PN n=8 | 1.0 | 38.9±22.0 | p=0.002 |

The p-values refer to comparisons between control and treated groups. Analysis was performed using a one-tail Student t-test. Similar values were obtained using non-parametric statistical analyses. All other values are mean ± SD for number of animals indicated.

It can be seen from Table 1 that when given at 0.25 mg/kg/d PN inhibited lesion development by 26% (Expt. 1). Increasing the dose to 1.0 mg/kg/d resulted in 46% inhibition, whereas PY inhibited 19% at a similar dose (Expt. 2). Vessel diameters (determined from cross-sections of perfusion fixed vessels) were comparable in all three experiments.

This effect appears to be independent of blood pressure lowering effects or platelet involvement.

An indicated daily dosage of a compound of formula I is in the range of from about 0.2 to about 350 mg, preferably 1 to 70 mg, especially 1 to 10 mg i.v., and from about 2 to 2000 mg per os or from about 1 to about 200 mg sublingually for interval therapy. Preferred for PY and PN is a daily dosage of from about 0.2 mg to about 10 mg i.v., from about 2 to about 50 mg per os or from about 1 to about 50 mg sublingually. Especially for interval therapy the unit dosage may be administered in divided dosages e.g. 3 times a day containing from about 0.1 to about 150 mg i.v. or about 0.7 to about 700 mg per os or about 0.3 to about 70 mg sublingually of the compound admixed with a solid pharmaceutical carrier or diluent.

The invention provides the use of a compound of formula I in the manufacture of a pharmaceutical composition for use in the treatment of atherosclerosis and for prevention of plaque formation in atherosclerosis.

The compounds of formula I may be administered on their own or in the form of pharmaceutical compositions.

Such composition conveniently contain more than 1% by weight of the compound of formula I and may be prepared by conventional techniques to be in conventional forms, for example capsules, tablets, suppositories, dispersible powders, suspension, for enteral or parenteral administration. Suitable pharmaceutical

diluents or carriers include, for example, alcohols, e.g. polyethyleneglycol, polyvinylpyrrolidone, mannitol and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan mono-oleate, granulating and disintegrating agents such as starch and alginic acid, binding agents such as starch, gelatine and acacia, and lubricating agents such as magnesium stearate, stearic acid and talc, in order to provide an elegant and palatable pharmaceutical preparation.

In view of the low solubility of many of the compounds of formula I it is preferred to employ solid forms. It is preferred to use pharmaceutical compositions formulated to facilitate rapid absorption of a compound of formula I. For example oral pharmaceutical compositions may be employed and formulated to dissolve rapidly in the mouth, e.g. sub-lingual tablets and capsules. Alternatively the pharmaceutical composition may be in powder or liquid form for administration as a spray or mist into the oral cavity.

The following Examples are illustrative of compositions for use in the invention.

**Example 1:**

**Hard gelatine capsules for oral administration**

Hard gelatine capsules containing the ingredients indicated below may be prepared by conventional techniques, and be administered once a day for the treatment of conditions related directly or indirectly to ionized calcium levels in the blood:

| Ingredient | Weight |
|---|---|
| PN | 10.0 mg |
| Polyvinylpyrrolidone | 30.0 mg |
| Lactose | 148.5 mg |
| Corn starch | 60.0 mg |
| Magnesium stearate | 1.5 mg |
| | 250.0 mg |

**Example 2:**

**Tablets for sublingual administration**

| Ingredient | Weight |
|---|---|
| PN | 10.0 mg |
| Polyethylenglycol 6000 | 33.0 mg |
| Mannitol | 50.0 mg |
| Polyvinylpyrrolidone | 4.5 mg |
| Talc | 2.0 mg |
| Magnesium stearate | 0.5 mg |
| | 100.0 mg |

**Example 3:**

**Soft gelatine capsules for sublingual administration**

| Ingredient | Weight |
|---|---|
| PN<br>Polyethyleneglycol 2000<br>Polyethyleneglycol 400 | 10.0 mg<br>100.0 mg<br>140.0 mg<br>250.0 mg |

Sufficient amounts of the above components are mixed in conventional manner and filled into gelatine capsules or compressed to tablets, which are administered once a day for the treatment of conditions related directly or indirectly to ionized calcium levels in the blood.

## Claims

**1.** Use of a compound of formula I

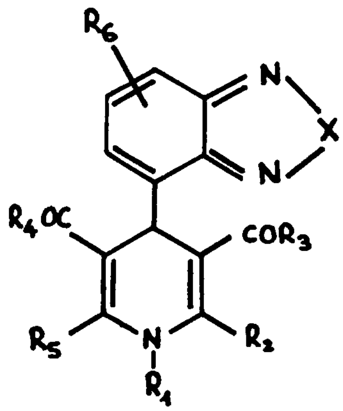

wherin

| | |
|---|---|
| $R_1$ | is hydrogen |
| $R_2$ and $R_5$ | independently are alkyl or 1 to 6 carbon atoms |
| $R_3$ and $R_4$ | independently are alkoxy of 1 to 6 carbon atoms |
| $R_6$ | is hydrogen |
| X | is oxygen |

in the preparation of a medicament suitable for use in the treatment of atherosclerosis.

**2.** Use of a compound of formula I according to claim 1 in the preparation of a medicament suitable for use in the prevention or inhibition of plaque formation in atherosclerosis.

**3.** Use of 4-(2,1,3-Benzoxadiazol-4-yl)-1,4-dihydro-2,6-dimethyl-3-methoxycarbonyl-pyridine-5-carboxylic acid isopropyl ester as compound of formula I in the preparation of a medicament according to claim 1 or 2.

**4.** Use of 4-(2,1,3-Benzoxadiazol-4-yl)-1,4-dihydro-2,6-dimethylpyridine-3,5-dicarboxylic acid diethylester as compound of formula I in the preparation of a medicament according to claim 1 or 2.

## Revendications

**1.** L'utilisation d'un composé de formule I

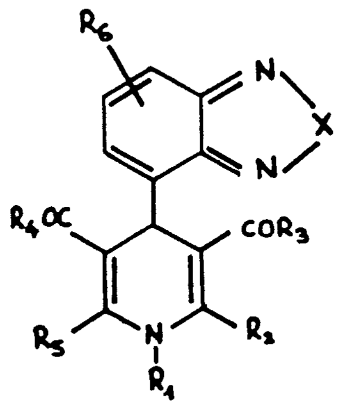

dans laquelle

- $R_1$ signifie l'hydrogène,
- $R_2$ et $R_5$ signifient indépendamment alkyle en $C_1$-$C_6$,
- $R_3$ et $R_4$ signifient indépendamment alcoxy en $C_1$-$C_6$,
- $R_6$ signifie l'hydrogène,
- X signifie l'oxygène,

pour la préparation d'un médicament approprié pour l'utilisation dans le traitement de l'athérosclérose.

**2.** L'utilisation d'un composé de formule I selon la revendication 1, pour la préparation d'un médicament approprié pour l'utilisation dans la prévention ou l'inhibition de la formation de plaques dans l'athérosclérose.

**3.** L'utilisation de l'ester isopropylique de l'acide 4-(2,1,3-benzoxadiazole-4-yl)-1,4-dihydro-2,6-diméthyl-3-méthoxycarbonyl-pyridine-5-carboxylique comme composé de formule I pour la préparation d'un médicament selon la revendication 1 ou 2.

**4.** L'utilisation de l'ester diéthylique de l'acide 4-(2,1,3-benzoxadiazole-4-yl)-1,4-dihydro-2,6-diméthyl-pyridine-3,5-dicarboxylique comme composé de formule I pour la préparation d'un médicament selon la revendication 1 ou 2.

## Patentansprüche

**1.** Verwendung einer Verbindung der Formel I

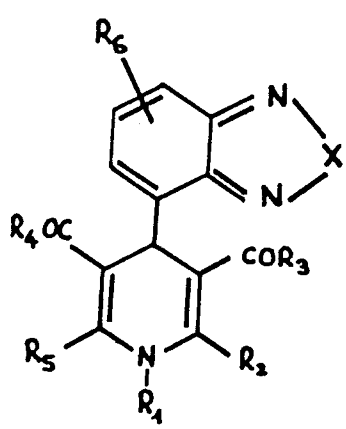

worin

- $R_1$ Wasserstoff
- $R_2$ und $R_5$ unabhängig voneinander Alkyl mit 1 bis 6 Kohlenstoffatomen
- $R_3$ und $R_4$ unabhängig voneinander Alkoxy mit 1 bis 6 Kohlenstoffatomen

| | |
|---|---|
| $R_6$ | Wasserstoff und |
| X | Sauerstoff bedeuten |

zur Herstellung eines Arzneimittels das zur Behandlung von Atherosclerose geeignet ist.

**2.** Verwendung einer Verbindung der Formel I gemäss Anspruch 1 zur Herstellung eines Arzneimittels das zur Vorbeugung oder Hemmung der Plaquebildung bei der Atherosclerose geeignet ist.

**3.** Verwendung von 4-(2,1,3-Benzoxadiazol-4-yl)-1,4-dihydro-2,6-dimethyl-3-methoxycarbonyl-pyridin-5-carbonsäure isopropyl ester als Verbindung der Formel I zur Herstellung eines Medikamentes gemäss den Ansprüchen 1 oder 2.

**4.** Verwendung von 4-(2,1,3-Benzoxadiazol-4-yl)-1,4-dihydro-2,6-dimethyl-pyridin-3,5-dicarbonsäure diethylester als Verbindung der Formel I zur Herstellung eines Medikamentes gemäss den Ansprüchen 1 oder 2.